# EUROPEAN PATENT SPECIFICATION

(11) **EP 4 243 779 B1**
(45) Date of publication and mention of the grant of the patent: **31.12.2025**
(21) Application number: 21865370.7
(22) Date of filing: 16.11.2021
(51) Int. Cl.: A61K 9/48, A61K 9/16, A61K 31/216

(54) **PHARMACEUTICAL GRANULATE FORMULATION AND METHOD FOR THE PREPARATION THEREOF**
PHARMAZEUTISCHE GRANULATFORMULIERUNG UND VERFAHREN ZU IHRER HERSTELLUNG
FORMULATION DE GRANULÉ PHARMACEUTIQUE ET SON PROCÉDÉ DE PRÉPARATION

(30) Priority: 16.11.2020 NL 2026905
(43) Date of publication of application: 20.09.2023
(73) Proprietor: PHARMAMATCH B.V., 1083 BA Amsterdam (NL)
(72) Inventor: VEMULA, Sathyanarayana, Mumbai 400705 (IN)
(74) Representative: Wittop Koning, Tom Hugo
(86) International application number: PCT/NL2021/050703
(87) International publication number: WO 2022/103269

(56) References cited:
- EP-A1- 2 918 268
- EP-B1- 3 256 107
- WO-A1-01/97803

## Description

The invention relates to a method for the preparation of pharmaceutical granulate formulation comprising racecadotril as active ingredient, a disintegrant, a hydrophilic excipient, a glidant and a lubricant as defined in claim 1, to a pharmaceutical granulate composition as defined in claim 8, and to a hard gelatine capsule comprising the said granulate, as defined in claim 9.

Racecadotril (C₂₁H₂₃NO₄S, Mᵣ = 385.5 g/mol), (±)-N-[2-[(acetylthio)methyl]-I-oxo-3-phenylpropyl]glycine phenylmethyl ester, also known as acetorphan is an antidiarrheal prodrug. The active metabolite thiorphan acts as a peripheral enkephalinase inhibitor. It has an antisecretory effect in that it reduces the secretion of water and electrolytes into the intestine, and racecadotril is used to treat acute diarrhea in both children and adults. Racecadotril is a synthetic dipeptide having the following formula:

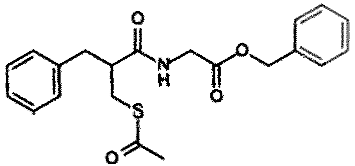

Racecadotril has a very low wettability and solubility in water which has a negative effect on the bioavailability. EP3256107 describes a method for dry granulating a mixture of racecadotril, a hydrophilic excipient and a starch-based disintegrant involving a high-pressure slugging compaction step using a round biconvex punch with a diameter of 7 mm and a bending radius of 9 mm at a compaction strength of 4 to 20 kN, i.e. a pressure of about 150 to 1,300 bar. A granulate is obtained having not more than 50 w/w% particles of a size below 90 µm. The said granulate has dissolution profile of 54, 68, 76, 86 and 93% after 10, 15, 20, 30 and 45 minutes, respectively.
The present inventors have now surprisingly found a granulate of the above kind with similar bioavailability and is thereto characterized in that the formulation contains the ingredients as further defined in claim 1 and is obtained by roller compaction with a compaction strength of 30 - 70 bar, wherein less than 1 w/w% of the granulate has a particle size of above 0.85 mm, 65 - 75 w/w% has a particle size of 0.25 - 0.85 mm, 30 - 50 w/w% of the particles have a particle size of 0.425 - 0.85 mm, 20 - 40 w/w% of the particles have a particle size of 0.25 - 0.425 mm, less than 30 w/w of the granulate has a particle size of below 0.25 mm, 10 - 20 w/w% of the granulate has a particle size of 0.165 - 0.25 mm, and less than 5 w/w% has a particle size of below 0.15 mm. It was found that such a granulate can effectively be prepared by roller compaction. EP3256107 concludes that finer particle size distributions correspond to faster dissolution kinetics and therefore aims at obtaining smaller particles. Surprisingly, the granulate of the invention, although being compacted at far less compaction strength, has a significantly coarser particle size distribution as that of the granulate of EP3256107, but still has similar dissolution kinetics.

Herein, the particle size are derived from mesh sizes and for that reason can be expressed to one thousandth of a mm. So herein, e.g. 0.25 mm should preferably be read as 0.250 mm, or as 250 µm.

Racecadotril granulates obtained by compaction are also described e.g. in WO01/97803 and EP2198268.

In the granulate of the invention, the relative content of small-sized granules, i.e. below 0.25 mm is rather low

Preferably, less than 0.5 w/w% and even more preferably, the granulate formulation is free of particles having a particle size of above 0.85 mm. Herein, the particle size is defined by sieving using a sieve of the corresponding mesh size. Particles with a particle size of above 0.85 mm will not pass a sieve with openings of 0.85 mm (i.e. 20-mesh). Particles with a particle size below e.g. 0.25 mm will pass a sieve with openings of 0.25 mm (i.e. 60-mesh).

The pharmaceutical granulate comprises
- 40 - 50 w/w% racecadotril,
- 30 - 40 w/w% pregelatinized starch having a moisture content of below 10 w/w%,
- 15 - 25 w/w% lactose monohydrate,
- 1.0 - 3.5 w/w% glidant, chosen from the group consisting of colloidal silicon dioxide, magnesium trisilicate, talc, tribasic calcium phosphate, calcium silicate and magnesium silicate, and
- 0.4-2.5w/w% lubricant, chosen from the groujp consisting of magnesium stearate, aluminium stearate, calcium stearate or zinc stearate.

It was found that such compositions result in the envisaged bioavailability.

The disintegrant is pregelatinized starch. The moisture content of the disintegrant is below 10 w/w%, preferably below 9 w/w% and most preferably below 8 w/w%.

The hydrophilic excipient is lactose monohydrate. The skilled person will be aware of suitably inorganic salts that can be used for incorporation in a pharmaceutical composition, such as e.g. sodium chloride.

The glidant is chosen from the group, consisting of: colloidal silicon dioxide, magnesium trisilicate, talc, tribasic calcium phosphate, calcium silicate and magnesium silicate. A preferred glidant is colloidal silicon dioxide.

The lubricant is a metal stearate, in particular magnesium stearate, aluminium stearate, calcium stearate or zinc stearate. A preferred lubricant is magnesium stearate.

Whenever reference is made in the specification to a compaction pressure in the unit of bar, it is understood that 1 bar is 100,000 Pascal.

The method comprises a roller compaction step with a compaction pressure of 30 - 70 bar. The method is preferably liquid-free, i.e. the ingredients are treated as dry components. As indicated above, a relatively low-pressure compaction by roller compaction within the indicated ranges result in a granulate as described above having the desired bioavailability.

In a particular embodiment, the said method comprises the steps of:
a) co-sieving the racecadotril, the pregelatinized starch, the lactose monohydrate, and the glidant through a 1.0 - 0.7 mm sieve,
b) mixing the sieved components of step a) to obtain a sieved mixture
c) sieving the lubricant through a 0.15 - 0.40 mm sieve,
d) adding 30 - 70% of the lubricant of step c) to the sieved mixture of step b) to obtain a blend, and
e) compacting the blend obtained in step d) using a roller compactor to obtain compacted material.

In step a), the ingredients except for the lubricant are combined and sieved through a sieve having the mesh size of the envisaged maximum particle size between 1.0 mm (18-mesh) and 0.7 mm (25-mesh). The said mesh size is preferably 0.85 mm, therewith removing particles larger than 0.85 mm. The sieving is followed by mixing the sieved components. Sieving can also be performed such, that the ingredients are mixed by the sieving process, therewith combining steps a) and b) in a single step.

In a separate step c), the lubricant is sieved through a sieve having the mesh size of the envisaged maximum particle size between 0.15 mm (150-mesh) and 0.40 mm (about 40-mesh), preferably of 0.25 mm (60-mesh).

In step d) 30 - 70 % of the lubricant sieved in step c) is added to the sieved mixture of the other ingredients as obtained ins step b). Preferably, 40 - 60 %, more preferably 50% of the lubricant is added in step c).

The blend obtained is then compacted using a roller compactor to obtain the compacted material. The pressure of the rollers is between 30 and 70 bar. The remainder of the lubricant is added after the compaction step.

In a preferred embodiment, the method further comprisies the following steps of:
f) sieving the compacted material of step e) through a 0.85 mm sieve,
g) sieving the material passed through the 0.85 mm sieve of step f) through a 0.25 mm sieve, yielding a retainer fraction having a particle size of 0.25 - 0.85 mm and a passed fraction having a particle size of below 0.25 mm,
h) collecting the retainer fraction and the passed fraction of step g), and
i) preparing a granulate mixture by combining at least a portion of the collected retainer fraction and at least a portion of the collected passed fraction, the granulate mixture comprising 50 - 70 w/w% of the retainer fraction and 30 - 50 w/w% of the passed fraction

The compacted material, obtained in step e) above is preferably sieved through a 0.85-mesh sieve in order to separate the coarser particles larger than 0.85 mm. The material passing the said sieve is then sieved through a 0.25 mm sieve, yielding a retainer fraction having the envisaged particle size of between 0.85 and 0.25 mm, whereas the material passing the 0.25 mm sieve has a smaller particle size. Both the retainer and passed fraction are collected and a granulate mixture of the invention can be prepared by combining the appropriate ratios of both fractions. It is also possible to use other cut-off particle sizes to arrive at a granulate mixture of an alternative particle size distribution. For example, the 0.85-mesh sieve can be replaced by any sieve in the range of e.g. 1.0 - 0.7 mm, and the 0.25 mm sieve can be replaced by a sieve in the range of e.g. 0.15 - 0.4 mm. however, the 0.85 mm and 0.25 mm sieves are preferred.

In an attractive embodiment, step f) comprises the steps of:
i. collecting the material retained on the 0.85 mm sieve,
ii. milling the material collected in step i., and
iii. recycle the milled material of step ii) to the sieving step f).

By including this loop of steps i. - iii., the coarse material retained on the sieve in step f) is milled to decrease the particle size of this fraction. After milling, this milled material is subjected to a further step f) to separate the particles that are still above the cut-off value, i.e. 0.85 mm, and to allow the smaller particle to be incorporated in the envisaged granulate. The loop of steps i. - iii can be repeated one or more times if desired.

In another embodiment, step h) further comprises the steps of:
I. compacting at least a portion of the passed fraction of step g) using a roller compactor, and
II. recycle the compacted material of step I. to the sieving step f).

By including this loop of steps I. - II., the fines that pass through the fine sieve of 0.25 mm in step g) is subjected to another compaction step, preferably under the same pressure conditions as the earlier compaction step e) in order to provide for larger particles that can be entered in the sieving step f). This embodiment is in particular desirable in case the fines content, i.e. having a particle size below the envisaged value of e.g. 0.25 mm is relatively high as compared to the envisaged portion having the envisaged particle size of between 0.25 and 0.85 mm. The loop of steps I. - II. can be repeated one or more times if desired.

As a last step, i.e. after the last compaction step but before the granulate mixture is e.g. packed in e.g. hard-gelatine capsules, the remainder of the lubricant is added to the granulate mixture of step i), or, if only steps a) to e) are performed, to the compacted material of step e).

The invention also relates to a pharmaceutical granulate composition as described above and to a hard gelatine capsule comprising the pharmaceutical granulate, in particular as prepared with the method as described above.

The invention will now be further illustrated by way of non-limiting examples.

### Ingredients:

Racecadotril: Symed Labs Ltd., India
Lactose monohydrate (Pharmatose 200M), DFE Pharma, Germany
Pregelatinized starch: Starch 1500, Colorcon, US
Colloidal silicon dioxide: Aerosil 200, Evonik, Germany
Magnesium stearate: Nitika Chemicals, India
Hard-gelatine Capsules: ACG, India
Composition: The relative amounts of the dry ingredients are given in table 1.

**Table 1: ingredients**

| Ingredients | kg |
|---|---|
| Racecadotril | 20.0 |
| Lactose monohydrate | 8.2 |
| Pregelatinized starch | 15.0 |
| Colloidal silicon dioxide | 1.0 |
| Magnesium stearate | 0.8 |

### Preparation:

### Ambient conditions: 22 ± 3°C, 55% humidity

The racecadotril, lactose monohydrate, the pregelatinized starch and the colloidal silicon dioxide were combined to a first mix and co-sieved through a 20-mesh sieve (vibrosifter) and blended in a pillar type bin blender (300 I.) for 10 minutes at 10 rpm at ambient conditions temperature.

The magnesium stearate was sieved separately through a 60-mesh sieve (vibrosifter). Half of the amount (0.4 kg) was added to the first mix and blended for 5 minutes in the pillar type bin blender (300 I.) at 10 rpm to give a second mix.

The second mix was subjected to roller compaction in a roller compactor (Alexanderwerk, Germany) fitted with a 1.0 mm screen. The process parameters are given in table 2.

**Table 2: Process parameters compaction**

| | |
|---|---|
| Roller Gap | 1.5 - 3.0 mm |
| Roller speed | 8.8 - 12 rpm |
| Feeder speed | 80 - 100 rpm |
| Hydraulic pressure | 60 - 70 bar |
| Pre-granulator | 50 - 150 rpm |
| Fine granulator | 50 - 150 rpm |

The granules collected from the 1.0 mm screen fine granulator compacted material was sieved through a 20-mesh sieve. The retainer was milled through a Quadro co-mill fitted with a 813µm SS screen and sieved through a 20-mesh screen.

The material that passed the 20-mesh sieve was sieved through a 60-mesh sieve, and the retainer material of the 60-mesh sieve was collected. The material that passed the 60-mesh sieve was subjected to roller compaction as described above. A granulate having 55 - 65 w/w% of the 60-mesh retainer material and the remainder of the 60-mesh passed material was made and blended for 3 minutes in a pillar type bin blender (150 I), whereafter the remaining 0.4 kg of the sieved magnesium stearate was added, followed by an additional blending of 5 minutes.

Quantities of 225 mg comprising 100 mg racecadotril were encapsulated in a Sejong automatic capsule filling machine at ambient temperature and 45% humidity.

| Bulk density: | |
|---|---|
| Bulk density (untapped): | 0.63 g/ml |
| Bulk density (tapped): | 0.83 g/ml |

### Particle size analysis:

The particle size was determined by sieve analysis. Table 3 shows the cumulative retention on ASTM sieves of 20-, 40-, 60-, 80- and 100-mesh (0.85, 0.425, 0.25, 0.165 and 0.149 mm, respectively. The fraction of 0.25 - 0.85 mm (20 - 60-mesh) constitutes about 73 w/w%, and below 0.25 mm 27 w/w%. In particular, it can be seen in table 3 that 42 w/w% of the particles have a size of 0.425 - 0.85 mm, 31% of the particles have a size of 0.25 - 0.425 mm, 17 w/w% of the particles have a size of 0.165 - 0.25 mm, and 6.6 w/w% of the particles have a size of 0.149 - 0.165 mm, whereas 3.4 w/w% of the particles are smaller than 0.149 mm.

**Table 3: Particle size**

| Particle size | w/w% |
|---|---|
| #20 | <1.0 |
| #40 | 41.9 |
| #60 | 53.5 |
| #80 | 63.7 |
| #100 | 67.2 |
| Receiver | 30.9 |
| Total | 98.1 |

### Dissolution profiling

Dissolution was performed with a USP type II (paddle with Japanese basket sinker) according to the instructions of the manufacturer in 900 ml 2.0% w/v sulphate pH 6.8 at a temperature of 37°C at a rotating speed of 100 rpm. The release percentages after 10, 15, 20, 30, 45 and 60 minutes are given in table 4.

The dissolution values as obtained are similar to those as obtained for Tiorfan^{®} and the compacted granulate of EP3256107.

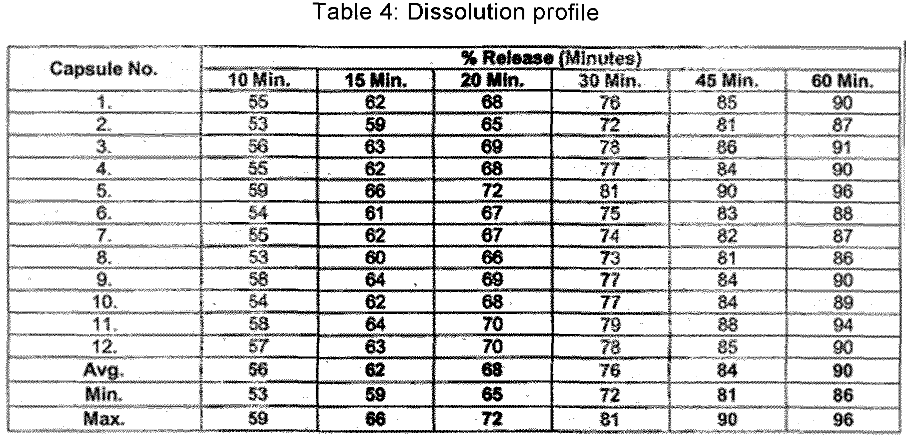

It was observed that the compaction pressure between the rollers could be varied between 20 - 100 bar. Similar results as described above were obtained with the claimed pressure between 30 and 70 bar.

## Claims

1. A method for the preparation of a pharmaceutical granulate formulation, the formulation comprising
- 40 - 50 w/w% racecadotril as active ingredient,
- 30 - 40 w/w% pregelatinized starch having a moisture content of below 10 w/w%,
- 15 - 25 w/w% lactose monohydrate,
- 1.0 - 3.5 w/w% glidant, chosen from the group, consisting of colloidal silicon dioxide, magnesium trisilicate, talc, tribasic calcium phosphate, calcium silicate and magnesium silicate, and
- 0.4 - 2.5 w/w% lubricant, chosen from the group, consisting of magnesium stearate, aluminium stearate, calcium stearate or zinc stearate,
**characterized in that** the method comprises a roller compaction step with a compaction strength of 3,000 kPa - 7,000 kPa (30 - 70 bar), less than 1 w/w% of the granulate having a particle size of above 0.85 mm, 65 - 75 w/w% having a particle size of 0.25 - 0.85 mm, 30 - 50 w/w% of the particles having a particle size of 0.425 - 0.85 mm, 20 - 40 w/w% having a particle size of 0.25 - 0.425 mm, less than 30 w/w having a particle size of below 0.25 mm, 10 - 20 w/w% having a particle size of 0.165 - 0.25 mm, less than 5 w/w having a particle size of below 0.15 mm.

2. The method of claim wherein the moisture content of the pregelatinized starch is below 9 w/w%, preferably below 8 w/w%.

3. The method of claim 1 or 2, comprising the steps of:
a) co-sieving the racecadotril, the pregelatinized starch, the lactose monohydrate, and the glidant through a 1.0 - 0.7 mm sieve,
b) mixing the sieved components of step a) to obtain a sieved mixture,
c) sieving the lubricant through a 0.15 - 0.40 mm sieve,
d) adding 30 - 70% of the lubricant of step c) to the sieved mixture of step b) to obtain a blend, and
e) compacting the blend obtained in step d) using a roller compactor to obtain compacted material.

4. The method of claim 3 or 4, further comprising the following steps of:
f) sieving the compacted material of step e) through a 0.85 mm sieve,
g) sieving the material passed through the 0.85 mm sieve of step f) through a 0.25 mm sieve, yielding a retainer fraction having a particle size of 0.25 - 0.85 mm and a passed fraction having a particle size of below 0.25 mm,
h) collecting the retainer fraction and the passed fraction of step g), and
i) preparing a granulate mixture by combining at least a portion of the collected retainer fraction and at least a portion of the collected passed fraction, the granulate mixture comprising 50 - 70 w/w% of the retainer fraction and 30 - 50 w/w% of the passed fraction.

5. Method of claim 4, wherein step f) further comprises the steps of:
i. collecting the material retained on the 0.85 mm sieve,
ii. milling the material collected in step i., and
iii. recycle the milled material of step ii) to the sieving step f).

6. The method of claim 4 or 5, wherein step h) further comprises the steps of:
I. compacting at least a portion of the passed fraction of step g) using a roller compactor, and
II. recycle the compacted material of step I. to the sieving step f).

7. Method of any of the claims 4 - 6, further comprising the step:
j) adding the remainder of the lubricant to the granulate mixture of step i).

8. A pharmaceutical granulate formulation, obtainable by the method of any of the preceding claims.

9. A hard gelatine capsule comprising the pharmaceutical granulate obtained by the method of any of claims 1 - 7 or the pharmaceutical granulate formulation of claim 8.

## Patentansprüche

1. Verfahren zur Herstellung einer pharmazeutischen Granulatformulierung, wobei die Formulierung umfasst
- 40 - 50 Gew.-% Racecadotril als Wirkstoff,
- 30 - 40 Gew.-% vorgelatinierte Stärke mit einem Feuchtigkeitsgehalt von unter 10 Gew.-%,
- 15 - 25 Gew.-% Lactose-Monohydrat,
- 1,0-3,5 Gew.-% Gleitmittel, ausgewählt aus der Gruppe bestehend aus kolloidalem Siliciumdioxid, Magnesiumtrisilicat, Talk, tribasischem Calciumphosphat, Calciumsilicat und Magnesiumsilicat, und
- 0,4-2,5 Gew.-% Schmiermittel, ausgewählt aus der Gruppe bestehend aus Magnesiumstearat, Aluminiumstearat, Calciumstearat oder Zinkstearat,
**dadurch gekennzeichnet, dass** das Verfahren einen Walzenverdichtungsschritt mit einer Verdichtungskraft von 3.000 kPA - 7.000 kPA (30 - 70 bar) umfasst, wobei weniger als 1 Gew.-% des Granulats eine Partikelgröße von über 0,85 mm aufweist, 65 - 75 Gew.-% eine Partikelgröße von 0,25 - 0,85 mm aufweist, 30 - 50 Gew.-% der Partikel eine Partikelgröße von 0,425 - 0,85 mm aufweist, 20 - 40 Gew.-% eine Partikelgröße von 0,25 - 0,425 mm aufweist, weniger als 30 Gew.-% eine Partikelgröße von unter 0,25 mm aufweist, 10 - 20 Gew.-% eine Partikelgröße von 0,165 - 0,25 mm aufweist, weniger als 5 Gew.-% eine Partikelgröße von unter 0,15 mm aufweist.

2. Verfahren nach Anspruch 1, wobei der Feuchtigkeitsgehalt der vorgelatinierten Stärke unter 9 Gew.-%, vorzugsweise unter 8 Gew.-%, liegt.

3. Verfahren nach Anspruch 1 oder 2, umfassend die folgenden Schritte:
a) gemeinsames Sieben von Racecadotril, vorgelatinierter Stärke, Lactose-Monohydrat und Gleitmittel durch ein 1,0 - 0,7 mm Sieb,
b) Mischen der gesiebten Komponenten aus Schritt a), um eine gesiebte Mischung zu erhalten,
c) Sieben des Gleitmittels durch ein 0,15 - 0,40 mm Sieb,
d) Zugeben von 30 bis 70 % des Gleitmittels aus Schritt c) zu der gesiebten Mischung aus Schritt b), um eine Mischung zu erhalten, und
e) Verdichten der in Schritt d) erhaltenen Mischung unter Verwendung eines Walzenverdichters, um verdichtetes Material zu erhalten.

4. Verfahren nach Anspruch 3, das ferner die folgenden Schritte umfasst:
f) Sieben des verdichteten Materials aus Schritt e) durch ein 0,85-mm-Sieb,
g) Sieben des durch das 0,85-mm-Sieb aus Schritt f) hindurchgegangenen Materials durch ein 0,25-mm-Sieb, wodurch eine Rückhaltefraktion mit einer Partikelgröße von 0,25 bis 0,85 mm und eine Durchgangfraktion mit einer Partikelgröße von weniger als 0,25 mm erhalten werden,
h) Sammeln der zurückgehaltenen Fraktion und der durchgelassenen Fraktion aus Schritt g) und
i) Herstellen einer Granulatmischung durch Kombinieren mindestens eines Teils der gesammelten zurückgehaltenen Fraktion und mindestens eines Teils der gesammelten durchgelassenen Fraktion, wobei die Granulatmischung 50 bis 70 Gew.-% der zurückgehaltenen Fraktion und 30 bis 50 Gew.-% der durchgelassenen Fraktion umfasst.

5. Verfahren nach Anspruch 4, wobei Schritt f) ferner die folgenden Schritte umfasst:
i. Sammeln des auf dem 0,85-mm-Sieb zurückgehaltenen Materials,
ii. Mahlen des in Schritt i. gesammelten Materials und
iii. Zurückführen des gemahlenen Materials aus Schritt ii) zum Siebschritt f).

6. Verfahren nach Anspruch 4 oder 5, wobei Schritt h) ferner die folgenden Schritte umfasst:
I. Verdichten mindestens eines Teils der durchgelassenen Fraktion aus Schritt g) unter Verwendung eines Walzenverdichters und
II. Rückführen des verdichteten Materials aus Schritt I. zum Siebschritt f).

7. Verfahren nach einem der Ansprüche 4 bis 6, das ferner den folgenden Schritt umfasst:
j) Hinzufügen des restlichen Schmiermittels zu der Granulatmischung aus Schritt i).

8. Eine pharmazeutische Granulatformulierung, erhältlich durch das Verfahren gemäß einem der vorstehenden Ansprüche.

9. Eine Hartgelatinekapsel, umfassend das pharmazeutische Granulat, erhalten durch das Verfahren gemäß einem der Ansprüche 1 bis 7, oder die pharmazeutische Granulatformulierung gemäß Anspruch 8.

## Revendications

1. Procédé de préparation d'une formulation pharmaceutique granulaire, la formulation comprenant
- 40 à 50 % en poids de racécadotril comme ingrédient actif,
- 30 à 40 % en poids d'amidon prégélatinisé ayant une teneur en humidité inférieure à 10 % en poids,
- 15 à 25 % en poids de lactose monohydraté,
- 1,0 à 3,5 % en poids de glissant, choisi dans le groupe constitué par le dioxyde de silicium colloïdal, le trisilicate de magnésium, le talc, le phosphate de calcium tribasique, le silicate de calcium et le silicate de magnésium, et
- 0,4 à 2,5 % en poids de lubrifiant, choisi dans le groupe constitué par le stéarate de magnésium, le stéarate d'aluminium, stéarate de calcium ou stéarate de zinc,
**caractérisé en ce que** le procédé comprend une étape de compactage au rouleau avec une force de compactage de 3 000 kPA à 7 000 kPA (30 à 70 bars), moins de 1 % en poids du granulat ayant une taille de particules supérieure à 0,85 mm, 65 à 75 % en poids ayant une taille de particules de 0,25 à 0,85 mm, 30 à 50 % en poids des particules ayant une taille de particules de 0,425 à 0,85 mm, 20 à 40 % en poids ayant une taille de particules de 0,25 à 0,425 mm, moins de 30 % en poids ayant une taille de particules inférieure à 0,25 mm, 10 à 20 % en poids ayant une taille de particules de 0,165 à 0,25 mm, moins de 5 % en poids ayant une taille de particules inférieure à 0,15 mm.

2. Procédé selon la revendication, dans lequel la teneur en humidité de l'amidon prégélatinisé est inférieure à 9 % en poids, de préférence inférieure à 8 % en poids.

3. Procédé selon la revendication 1 ou 2, comprenant les étapes suivantes:
a) tamisage conjoint du racécadotril, de l'amidon prégélatinisé, du lactose monohydraté et de l'anti-agglomérant à travers un tamis de 1,0 à 0,7 mm,
b) mélange des composants tamisés de l'étape a) pour obtenir un mélange tamisé,
c) tamisage du lubrifiant à travers un tamis de 0,15 à 0,40 mm,
d) ajouter 30 à 70 % du lubrifiant de l'étape c) au mélange tamisé de l'étape b) pour obtenir un mélange, et
e) compacter le mélange obtenu à l'étape d) à l'aide d'un compacteur à rouleaux pour obtenir un matériau compacté.

4. Procédé selon la revendication 3 ou 4, comprenant en outre les étapes suivantes:
f) tamisage du matériau compacté de l'étape e) à travers un tamis de 0,85 mm,
g) tamisage du matériau passé à travers le tamis de 0,85 mm de l'étape f) à travers un tamis de 0,25 mm, ce qui donne une fraction retenue ayant une taille de particules de 0,25 à 0,85 mm et une fraction passée ayant une taille de particules inférieure à 0,25 mm,
h) recueillir la fraction retenue et la fraction passée de l'étape g), et
i) préparer un mélange granulaire en combinant au moins une partie de la fraction retenue recueillie et au moins une partie de la fraction passée recueillie, le mélange granulaire comprenant 50 à 70 % en poids de la fraction retenue et 30 à 50 % en poids de la fraction passée.

5. Procédé selon la revendication 4, dans lequel l'étape f) comprend en outre les étapes suivantes:
i. collecter le matériau retenu sur le tamis de 0,85 mm,
ii. broyer le matériau collecté à l'étape i., et
iii. recycler le matériau broyé de l'étape ii) vers l'étape de tamisage f).

6. Procédé selon la revendication 4 ou 5, dans lequel l'étape h) comprend en outre les étapes consistant à:
I. compacter au moins une partie de la fraction passée de l'étape g) à l'aide d'un compacteur à rouleaux, et
II. recycler le matériau compacté de l'étape 1. vers l'étape de tamisage f).

7. Procédé selon l'une quelconque des revendications 4 à 6, comprenant en outre l'étape consistant à:
j) ajouter le reste du lubrifiant au mélange de granulés de l'étape i).

8. Une formulation pharmaceutique sous forme de granulés, pouvant être obtenue par le procédé de l'une quelconque des revendications précédentes.

9. Une capsule de gélatine dure comprenant les granulés pharmaceutiques obtenus par le procédé de l'une quelconque des revendications 1 à 7 ou la formulation pharmaceutique sous forme de granulés de la revendication 8.
